# EUROPÄISCHE PATENTSCHRIFT

(11) **EP 1 789 141 B1**
(45) Veröffentlichungstag und Bekanntmachung des Hinweises auf die Patenterteilung: **12.03.2008**
(21) Anmeldenummer: 05774992.1
(22) Anmeldetag: 05.08.2005
(51) Int. Cl.: A61Q 17/04, A61K 8/49

(54) **ZUSAMMENSETZUNG ENTHALTEND EINEN DIHYDROXYACETON-VORLÄUFER**
COMPOSITION CONTAINING A DIHYDROXYACETONE PRECURSOR
COMPOSITION CONTENANT UN PRECURSEUR DE DIHYDROXYACETONE

(30) Priorität: 01.09.2004 US 930778
(43) Veröffentlichungstag der Anmeldung: 30.05.2007
(73) Patentinhaber: Merck Patent GmbH, 64293 Darmstadt (DE)
(72) Erfinder: MUJICA-FERNAUD, Teresa, 64289 Darmstadt (DE); CAROLA, Christophe, 69120 Heidelberg (DE); HUBER, Sylvia, 64646 Heppenheim (DE); BUCHHOLZ, Herwig, 60599 Frankfurt (DE)
(86) Internationale Anmeldenummer: PCT/EP2005/008525
(87) Internationale Veröffentlichungsnummer: WO 2006/024361

(56) Entgegenhaltungen:
- US-A- 3 668 226
- US-A- 5 625 062
- US-A1- 2002 051 755

## Beschreibung

Die Erfindung betrifft eine kosmetische oder dermatologische Zusammensetzung enthaltend mindestens einen Vorläufer einer Substanz, deren aktive Form wegen ihrer kosmetische Wirkung gefragt ist. Eine der Anwendungen der Erfindung betrifft Zusammensetzungen, die in der Lage sind, der Haut schnell eine Farbe zu verleihen, die derjenigen ähnlich ist, die man durch längere Aussetzung an ultraviolette Sonnen- oder künstliche Bestrahlung erhält, wobei aber gleichzeitig die Nachteile einer solchen Aussetzung (Erythem, Verbrennung, Elastizitätsverlust, Auftreten von Falten, vorzeitige Hautalterung und ähnliches) vermieden werden.

Seit Jahren lehrt der Stand der Technik die Rolle von Dihydroxyaceton (im folgenden Text DHA) bei der künstlichen Färbung der Haut (Bobin et al., J. Soc. Cosmet. Chem. 35, Seite 265-272, 1984). DHA reagiert mit den Aminosäuren, die von Natur aus im Fettfilm der Hornschicht enthalten sind, und bildet über eine Maillard-Reaktion Melanoide (Maillard L. C., C. R. Acad. Sci. 154, 66-68, 1912).

Kosmetische Zusammensetzungen, die zur künstlichen Färbung der Haut dienen und DHA enthalten, sind im Stand der Technik ausführlich beschrieben.

Um den Effekt von DHA zu verbessern, wird es häufig mit anderen Substanzen kombiniert, damit die Schnelligkeit, mit der die Farbe erscheint, oder die Beständigkeit der letzteren über einen Zeitraum zunimmt. Hierbei sind beispielsweise die Kombinationen zu nennen, die in den Anmeldungen WO-A-9404130 oder EP-A-547 864 beschrieben sind.

Die Verwendung von DHA hat eine Reihe von Nachteilen, die mit Attraktivität für den Kunden kaum vereinbar sind. So ist die Stabilität von DHA in Formulierungen ausschließlich relativ, was im Laufe der Zeit zum Abbau der Verbindung führt. Insbesondere ist zu beobachten, dass Zusammensetzungen, die DHA enthalten, vor der Verwendung manchmal eine Farbe annehmen, die Anwender unangenehm finden. Außerdem kann sich bei diesen Zusammensetzungen im Laufe der Zeit ein Übelkeit erregender und unangenehmer Geruch entwickeln, was Kunden im Allgemeinen nicht wünschenswert finden. Der pH-Wert der Zusammensetzung nimmt im Laufe der Zeit ebenfalls ab, was die Zusammensetzung letztendlich für eine Verwendung in topischen Anwendungen ungeeignet macht.

Wenn man nur die Wirkung des DHA berücksichtigt, ist zudem bekannt, dass seine Remanenz auf der Haut nicht perfekt ist.

Um diesen Nachteilen abzuhelfen, schlägt US 5,693,670 eine neue kosmetische Zusammensetzung vor, bei der eines der Ziele darin besteht, DHA zum Zeitpunkt des Auftragens der letzteren, insbesondere auf die Haut, bereitzustellen, ohne dass jedoch die besagte Zusammensetzung DHA als solches enthält. Die kosmetische oder dermatologische Zusammensetzung enthält in einem kosmetisch oder dermatologisch verträglichen Träger mindestens ein verestertes Dihydroxyaceton-Derivat entsprechend der allgemeinen Formel: R-O-CH₂-C(=O)-CH₂-O-R', in der R und R' ein Wasserstoffatom oder einen gesättigten oder ungesättigten, linearen, verzweigten oder cyclischen, gegebenenfalls hydroxylierten Acylrest mit 2 bis 25 Kohlenstoffatomen bedeuten, wobei R und R' gleich oder verschieden sein können mit der Bedingung, dass sie nie gleichzeitig ein Wasserstoffatom bedeuten.

Obwohl diese DHA-Vorläufer synthetisiert wurden, besteht in der kosmetischen und dermatologischen Industrie noch immer ein Bedarf an neuen selbstbräunenden Verbindungen mit zusätzlichen Vorteilen.

Die vorliegende Erfindung betrifft die Verwendung in der Kosmetik und Dermatologie von Verbindungen der Formel I als Vorläufer des Dihydroxyacetons, insbesondere die Anwendung dieser Verbindungen als Selbstbräuner in kosmetischen und dermatologischen Zusammensetzungen. Die vorliegende Erfindung stellt kosmetisch und dermatologisch wirksame Zusammensetzungen bereit, die Verbindungen of Formel I 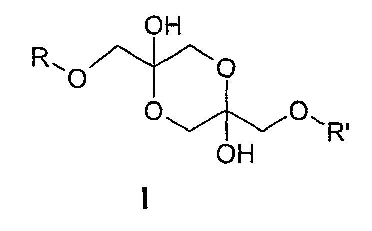 enthalten, in der R und R' ein Wasserstoffatom oder einen gesättigten oder ungesättigten, linearen, verzweigten oder cyclischen Acylrest mit 2 bis 25 Kohlenstoffatomen bedeuten, wobei R und R' gleich oder verschieden sein können unter der Bedingung, dass sie nie gleichzeitig ein Wasserstoffatom bedeuten.

Verbindungen der Formel I sind dimere Moleküle, die aus zwei kovalent gebundenen Monoester-Derivaten des Dihydroxyacetons (DHA) bestehen. Die Synthese der Verbindungen gemäß Formel I erfolgt unter Verwendung von Dihydroxyaceton und Fettsäuren oder Fettsäurechloriden. Es können Standardverfahren für die Kondensation eines Alkohols mit einer Säure oder einem Säurechlorid zur Herstellung von Estern gemäß Can. J. of Chem, 1969, 1249 - welches hierin durch Bezugnahme aufgenommen ist - verwendet werden.

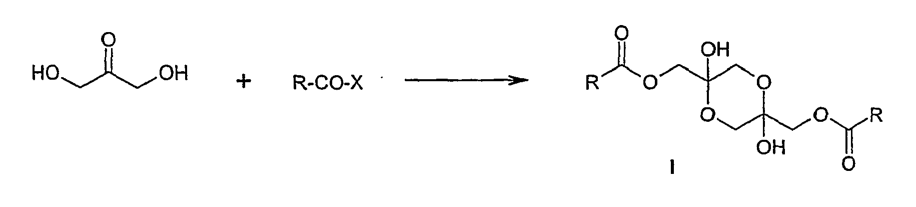

Unter den Reaktionsbedingungen können Verbindungen gemäß Formel I direkt hergestellt werden.

Längeres Erhitzen von I in ethanolischer Lösung ergibt die monomere Form, die sich durch Stehenlassen im kristallinen Zustand bei Raumtemperatur langsam wieder in I umwandelt.

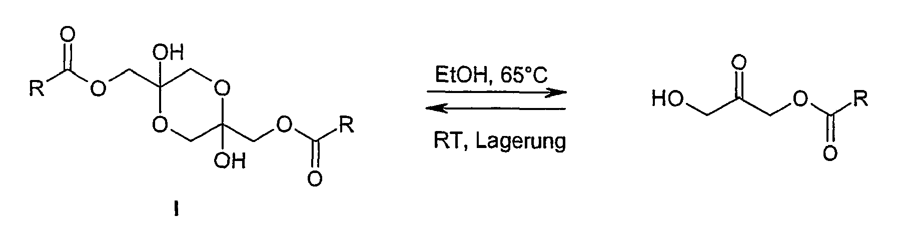

Die Vorteile einer Verwendung von Verbindungen gemäß Formel I oder Zusammensetzungen, die diese Verbindungen enthalten, sind unten zusammengefasst:
- Das Eindringen der Verbindungen I in die Haut ist durch die Anwesenheit von Fettsäureteilen verbessert.
- Verbindungen I werden durch in der Haut vorhandene Enzyme, die mit dem Fettstoffwechsel in Zusammenhang stehen, zu DHA hydrolysiert.
- Verbindungen I entwickeln eine Bräunung nicht so schnell wie DHA. Der Bräunungsvorgang dauert länger (-3 Tage). Die Kombination von DHA und I in Formulierungen sorgt dafür, dass die Bräune länger hält.
- Verbindungen I sind Feststoffe und leicht zu formulieren.

Die oben beschriebene Erfindung ermöglicht es, sowohl die Remanenz des aufgetragenen Produktes als auch seine Stabilität in Formulierungen zu verbessern. Trotzdem bleibt es so, dass einer der für eine solche kosmetische oder dermatologische Zusammensetzung gewünschten Hauptvorteile, nämlich die Verwendung von DHA wegen seiner Eigenschaften, nur erzielt werden kann, wenn das veresterte Derivat zum Zeitpunkt der Anwendung in DHA umgewandelt wird. Eine solche Hydrolyse, die zum Zeitpunkt der Anwendung DHA freisetzt, ist nur in Gegenwart von einer (oder mehreren) Verbindungen möglich, die zur Spaltung der Esterbindung(en) des Derivats befähigt sind. Solche Verbindungen finden sich auf der Haut. Um jedoch die Wirksamkeit der erfindungsgemäßen Zusammensetzung zu verbessern, ist es in einer Ausführungsform der Erfindung erwünscht, das veresterte DHA-Derivat und die zur Spaltung einer Esterbindung befähigte Verbindung gleichzeitig bereitzustellen.

Bevorzugte Zusammensetzungen enthalten daher mindestens eine zur Spaltung mindestens einer Esterbindung befähigte Verbindung.

Diese Zusammensetzung kann zum Zwecke ihrer Verwendung in den betreffenden Bereichen vorteilhaft in einer kosmetisch oder dermatologisch verträglichen Form vorliegen.

Nach einer bevorzugten Ausführungsform der Erfindung hat der Acylrest 3 bis 18 Kohlenstoffatome.

Das veresterte Derivat kann man vorzugsweise aus der Gruppe 1,3-Didodecansäure-2-oxopropylester, 1,3-Dihexadecansäure-2-oxopropylester, 3-Hexadecansäure-2-oxopropylester auswählen.

Nach einer bevorzugten Ausführungsform der Erfindung kann das veresterte Derivat in einer Konzentration im Bereich von 0,1 % bis 20% und vorzugsweise in einer Konzentration im Bereich von 0,5% bis 10% vorliegen.

Hier und im restlichen Text sind Prozente Gewichtsprozent, bezogen auf das Gesamtgewicht der Zusammensetzung.

Nach einer spezifischen Ausführungsform der letzteren kann die zur Spaltung mindestens einer Esterbindung befähigte Verbindung jede in der Kosmetik verträgliche nukleophile Verbindung sein. Zu nennen wären also Alkohole, Thiole, Amine oder Anionen.

Unter den Aminen wird vorzugsweise ein hydroxyliertes Amin, wie z.B. 3-Amino-1,2-propandiol, 2-Amino-2-methyl-1,3-propandiol, 2-Amino-2-methylpropanol, 2-Amino-2-hydroxymethyl-1,3-propandiol, Glucamin oder N-Methylglucamin, oder eine Aminosäure, wie z.B. Lysin, Arginin oder Histidin, ausgewählt.

Unter den Anionen wird vorzugsweise ein Carboxylatanion, wie z.B. Fettsäuresalze, Aminosäuresalze oder Lipoaminosäuresalze, ausgewählt.

Enzyme stellen die zweite große Familie von zur Spaltung mindestens einer Esterbindung befähigten Verbindungen, die erfindungsgemäß verwendet werden können. Beispiele, die zu nennen wären, sind Hydrolasen, unter denen Lipasen, Esterasen oder Proteasen zu nennen wären, ohne die Erfindung hierauf zu beschränken. Bei den Lipasen wird vorzugsweise eine pankreatische Schweinelipase, wie sie unter dem Namen Typ II von Sigma verkauft wird, oder anderenfalls die von Novo-Nordisk vertriebene Lipolase SP 644 ausgewählt.

Nach der zweiten Aufgabe der Erfindung und in einer weiteren speziellen Ausführungsform der letzteren kann man die zur Spaltung mindestens einer Esterbindung befähigte Verbindung in einer Konzentration im Bereich von 0,1% bis 30% und vorzugsweise im Bereich von 0,5% bis 15% verwenden.

In der normalen Anwendung solcher Zusammensetzungen wird man jedoch bevorzugt darauf abzielen, dass die Hydrolyse des veresterten DHA-Derivats erst zum Zeitpunkt der Anwendung der Zusammensetzungen stattfindet. Es ist daher von Vorteil, eine Verpackung bereitzustellen, in der das veresterte Derivat und die zur Spaltung mindestens einer Esterbindung befähigte Verbindung so verpackt sind, dass sie nicht miteinander in Kontakt stehen.

Eine weitere Aufgabe der Erfindung betrifft daher eine Zusammensetzung, bei der das veresterte Derivat und die zur Spaltung mindestens einer Esterbindung befähigte Verbindung so verpackt werden können, dass sie nicht miteinander in Kontakt stehen.

Die beiden getrennten, miteinander verbundenen Fächer können beispielsweise eine einzige Verpackung in Form einer flexiblen Tube darstellen, so dass das veresterte Derivat und die zur Spaltung mindestens einer Esterbindung befähigte Verbindung erst dann miteinander vermischt werden, wenn sie jeweils aus ihrem eigenen Fach herausgedrückt werden. Dieses Herausdrücken kann gleichzeitig oder nicht gleichzeitig stattfinden. Mischen findet während der Anwendung statt.

Eine andere Form einer Verpackung mit zwei getrennten, miteinander verbundenen Fächern kann so ausgestaltet sein, dass das veresterte Derivat oder die zur Spaltung einer Esterbindung befähigte Verbindung in Form von Mikrokapseln, Kügelchen oder einer anderen dem Fachmann bekannten Form eingekapselt und in Gegenwart der anderen erfindungsgemäßen Komponente in einer anderen Form, die dem Fachmann als solche bekannt ist, verpackt ist.

In einer kosmetischen oder dermatologischen Form dieser Verpackung kann es sich bei dem nicht verkapselten Teil der Erfindung um eine Creme, ein Gel oder eine beliebige andere dem Fachmann bekannte Form handeln.

Es gibt keinen Grund, warum nicht auch beide Komponenten jeweils verkapselt sein können. Auch hier ist dem Fachmann bekannt, wie solche Formen hergestellt werden.

Die Freisetzung findet während der Anwendung statt, indem der Benutzer Druck ausübt und die Kapseln zerdrückt, und die so freigesetzten Zusammensetzungen vermischen sich.

Unabhängig von der Ausführungsform der Erfindung kann sie in einer kosmetischen oder dermatologischen Anwendung auch beliebige andere kosmetisch oder dermatologisch verträgliche Bestandteile enthalten, die normalerweise in dieser Art von Zusammensetzung verwendet werden, und insbesondere Zusätze, die verwendet werden, um die Wirksamkeit des DHA, das aus der Hydrolyse des Derivats stammt, zu erhöhen.

Die vorliegende Erfindung stellt weiterhin Formulierungen enthaltend mindestens eine selbstbräunende Substanz gemäß Formel bereit, dadurch gekennzeichnet, dass die Formulierung mindestens einen fettigen Träger und mindestens ein hydrophiles Lösungsmittel enthält.

Für die Zwecke der vorliegenden Erfindung können die Formulierungen zusätzliche selbstbräunende Substanzen oder selbstbräunende Mittel enthalten. Geeignete zusätzliche selbstbräunende Substanzen oder selbstbräunende Mittel sind alle Substanzen oder Substanzgemische, die in der Lage sind, die menschliche Haut ohne die Einwirkung von UV-Strahlung zu bräunen. Vorteilhafte selbstbräunende Mittel, die man für die Zwecke der vorliegenden Erfindung als zusätzliche selbstbräunende Mittel verwenden kann, sind die folgenden Substanzen:

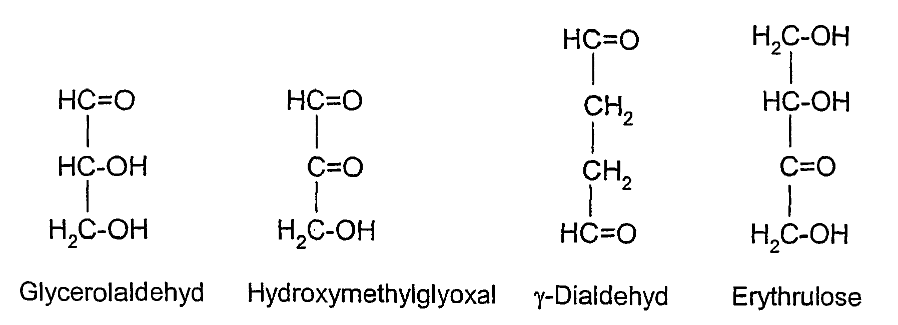

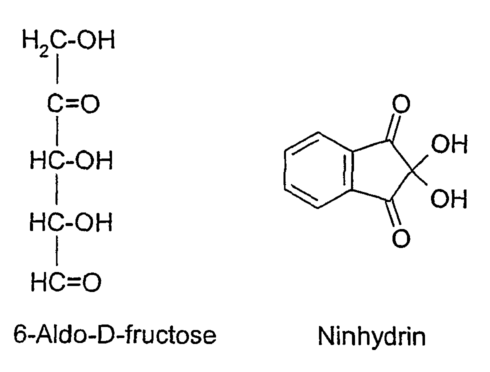

Ferner ist das 5-Hydroxy-1,4-naphthochinon (Juglon) zu nennen, das aus den Schalen frischer Walnüsse extrahiert wird 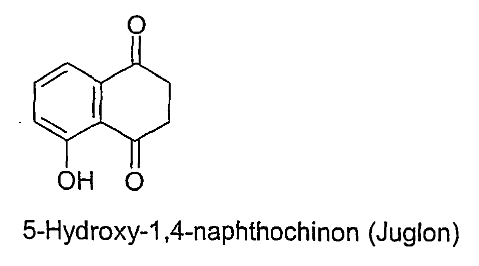 sowie das in Henna-Blättern vorkommende 2-Hydroxy-1,4-naphthochinon (Lawson).

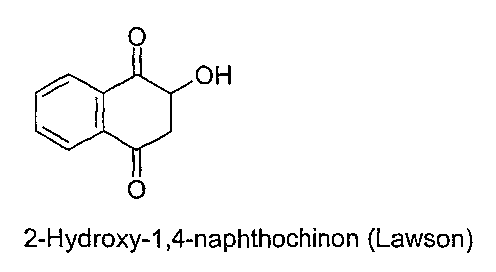

Der wichtigste Wirkstoff für die Selbstbräunung gemäß der vorliegenden Erfindung ist das 1,3-Dihydroxyaceton (DHA), ein im menschlichen Körper vorkommender dreiwertiger Zucker.

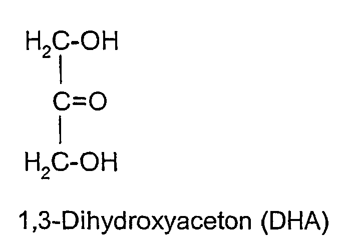

Die Konzentration der mindestens einen zusätzlichen selbstbräunenden Substanz, die erfindungsgemäß bevorzugt ist, liegt im Bereich von 0,01 bis 15 Gewichtsprozent, vorzugsweise im Bereich von 0,05 bis 5 Gewichtsprozent und besonders bevorzugt bei nicht mehr als 1 Gew.-%.

Der Zusatz hydrophiler Lösungsmittel erhöht die Bräunungsintensität. Infolgedessen kann man die Konzentration der selbstbräunenden Substanz weiter herabsetzen. Zudem sind die hydrophilen Lösungsmittel in der Lage, für eine gleichmäßigere Verteilung der selbstbräunenden Substanz zu sorgen, insbesondere wenn diese durch Versprühen aufgetragen wird.

Die erfindungsgemäß zu verwendenden hydrophilen Lösungsmittel können vorteilhaft aus den folgenden Substanzengruppen ausgewählt werden:
- Monoalkohole mit niedriger Kohlenstoffzahl, z.B. Isopropanol,
- mehrwertige Alkohole, wie vorzugsweise Propylenglykol oder Glycerol,
- Ester von Fettalkoholen mit Alkansäuren niedriger Kohlenstoffzahl.

Die erfindungsgemäß bevorzugten hydrophilen Lösungsmittel sind Propylenglykol und/oder Glycerol.

Die bevorzugte Konzentration an hydrophilen Lösungsmitteln, insbesondere Propylenglykol und/oder Glycerol, in erfindungsgemäßen Formulierungen liegt im Bereich von 0,1 to 20 Gewichtsprozent.

Zusätzlich sollte die Anwesenheit sogenannter fettiger Träger zu einer erhöhten Bräunungsintensität führen. Die erfindungsgemäß fettige Träger genannten Substanzen werden allgemein auch als "Schleuser" bezeichnet, da durch sie die Moleküle des selbstbräunenden Mittels in tiefere Schichten des Stratum corneums transportiert werden.

Bei den fettigen Trägern sind hier insbesondere Ceramide, Cholesterin, Phospholipide, Cholesterylsulfat, Cholesterylphosphat, Phosphatidylcholin, Lecithin und/oder Leerliposome zu nennen.

Erfindungsgemäß sind unter Phospholipiden die folgenden Substanzen zu verstehen: Phosphatidsäuren, die tatsächlichen Lecithine, Cardiolipine, Lysophospholipide, Lysolecithine, Plasmalogene, Phosphosphingolipide, Sphingomyeline. Bevorzugte Substanzen sind unten beschrieben.

Phosphatidsäuren sind Glycerol-Derivate, die in der 1-sn- und 2-Position mit Fettsäuren verestert sind (1-sn-Position: meist gesättigt, 2-Position: meist ein- oder mehrfach ungesättigt), am Atom 3-sn hingegen mit Phosphorsäure, und gekennzeichnet sind durch die allgemeine Strukturformel

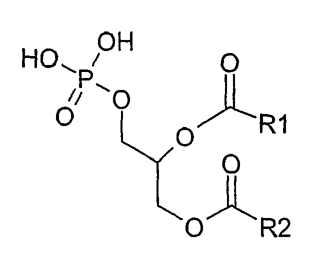

In den in menschlichem oder tierischem Gewebe vorkommenden Phosphatidsäuren ist der Phosphatrest meist mit Aminoalkoholen wie Cholin (Lecithin = 3-sn-Phosphatidylcholin) oder 2-Aminoethanol (Ethanolamin) oder L-Serin (Cephalin = 3-sn-Phosphatidylethanolamin oder sn-Phosphatidyl-L-serine), mit Myo-inositol zu den in Geweben häufig vorkommenden Phosphoinositiden [1-(3-sn-Phosphatidyl)-d-myoinositolen], mit Glycerol zu Phosphatidylglycerolen verestert. Besonderen Vorzug genießen die Lecithine (= 3-sn-Phosphatidylcholin).

Lecithine sind gekennzeichnet durch die allgemeine Strukturformel 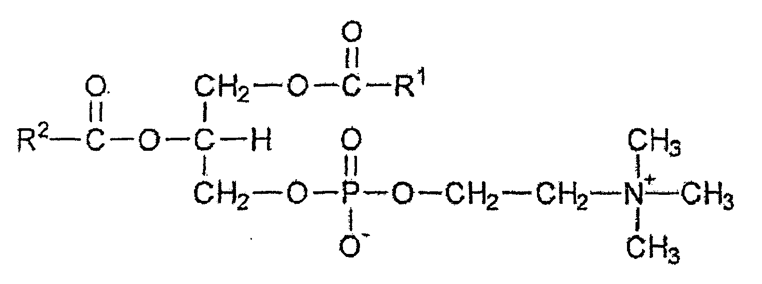 wobei R¹ und R² typischerweise unverzweigte aliphatische Reste mit 15 oder 17 Kohlenstoffatomen und bis zu 4 cis-Doppelbindungen bedeuten.

Cardiolipine (1,3-Bisphosphatidylglycerole) sind Phospholipide, die zwei über Glycerol gebundene Phosphatidsäuren enthalten.

Lysophospholipide erhält man, wenn ein Acylrest durch Phospholipase A (z.B. Lysolecithine) von Phospholipiden abgespalten wird. Lysophospholipide sind gekennzeichnet durch die allgemeine Strukturformel

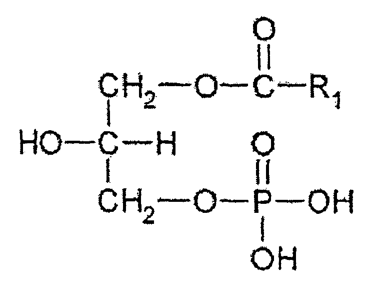

Lysolecithine sind beispielsweise gekennzeichnet durch die allgemeine Strukturformel 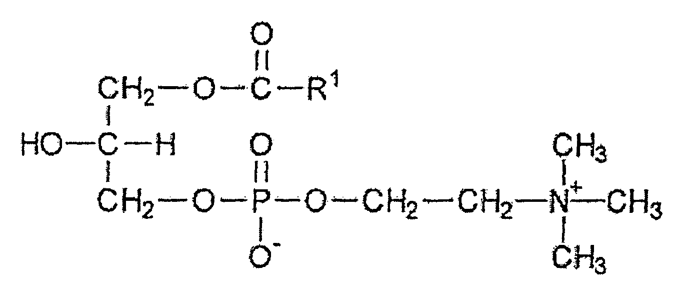 wobei R¹ typischerweise unverzweigte aliphatische Reste mit 15 oder 17 Kohlenstoffatomen und bis zu 4 cis-Doppelbindungen bedeutet.

Zu den Phospholipiden zählen auch die Plasmalogene, bei denen in der 1-Position statt einer Fettsäure ein Aldehyd (in Form eines Enolethers) gebunden ist; die O-1-sn-Alkenyl-Verbindungen, die den Phosphatidylcholinen entsprechen, werden beispielsweise Phosphatidalcholine genannt.

Als Grundstruktur bauen die Phosphosphingolipide auf Sphingosin oder auch Phytosphingosin auf, die gekennzeichnet sind durch die folgenden Strukturformeln:

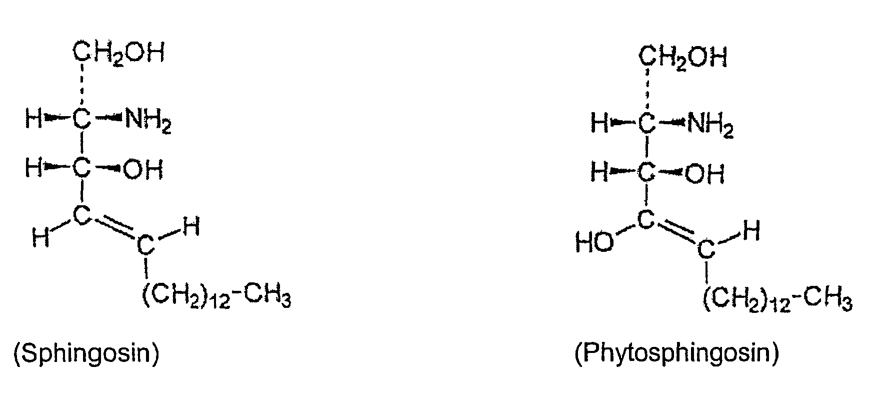

Modifikationen der Sphingolipide sind beispielsweise gekennzeichnet durch die allgemeine Grundstruktur 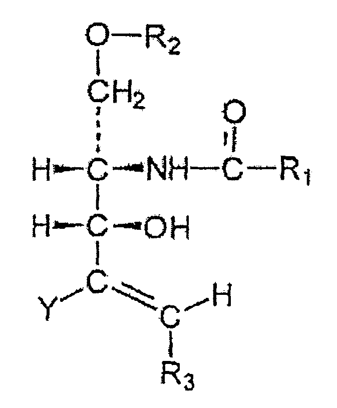 in der R₁ und R₃ unabhängig voneinander gesättigte oder ungesättigte, verzweigte oder unverzweigte Alkylreste mit 1 bis 28 Kohlenstoffatomen bedeuten, R₂ ausgewählt ist aus der Gruppe: Wasserstoffatom, gesättigte oder ungesättigte, verzweigte oder unverzweigte Alkylreste mit 1 bis 28 Kohlenstoffatomen, Zuckerresten, Phosphatgruppen, die unverestert oder mit organischen Resten verestert sind, Sulfatgruppen, die unverestert oder mit organischen Resten verestert sind, und Y entweder ein Wasserstoffatom, eine Hydroxygruppe oder einen anderen heterofunktionellen Rest bedeutet.

### Sphingophospholipide

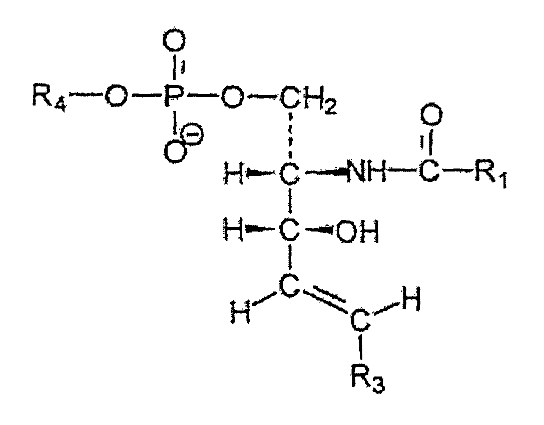

R₁ und R₃ stehen für Alkylreste, R₄ steht für einen Organylrest. Sphingomyeline sind organylphosphorylierte Sphingolipide des Typs

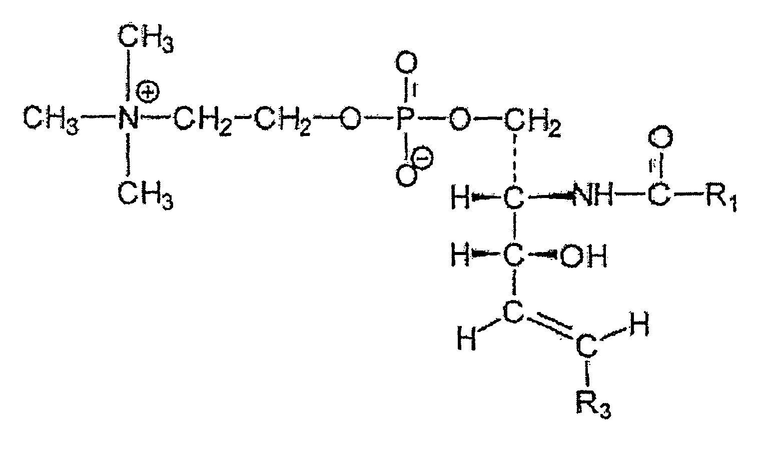

Besonders bevorzugte Phospholipide sind die Lecithine. Lecithintypen, die mit Vorteil zu verwenden sind, sind ausgewählt aus den Rohlecithinen, die entölt und/oder fraktioniert und/oder sprühgetrocknet und/oder acetyliert und/oder hydrolysiert und/oder hydriert wurden. Sie sind im Handel erhältlich. Bevorzugt werden Sojalecithine.

Erfindungsgemäß verwendet man vorteilhaft Ceramide, Cholesterin, Phospholipide, Fettsäuren, Cholesterylsulfat, Cholesterylphosphat, Phosphatidylcholin, Lecithin und/oder leere Liposome.

Erfindungsgemäß mit Vorteil zu verwendende Phospholipide sind käuflich beispielsweise unter den Handelsnamen Phospholipon 25 oder Phospholipon 90 (Nattermann), Emulmetik 120 (Lucas Meyer), Sternpur E (Stern), Sternpur PM (Stern), Nathin 3KE (Stern), Phospholipon 90 H (Nattermann/Rhone-Poulenc), Lipoid S 100 (Lipoid) erhältlich.

Die bevorzugte Konzentration an fettigen Trägern liegt erfindungsgemäß im Bereich von 0,1 bis 10 Gewichtsprozent.

Es ist erfindungsgemäß weiterhin bevorzugt, wenn die Formulierungen UV-Filter enthalten. Da auch diese UV-Filter beim Auftragen der Formulierung mit der Haut in Kontakt kommen, sollte es sich um UV-Filter handeln, die bei einer topischen Anwendung kompatibel sind. In diesem Zusammenhang tritt der zusätzliche Vorteil auf, dass diese UV-Filter beim Auftragen ebenfalls gleichmäßig durch die Haut absorbiert werden und die Haut somit gegen UV-Strahlung schützen.

Besonders bevorzugt sind solche UV-Filter, deren physiologische Unbedenklichkeit bereits nachgewiesen ist. Sowohl für UVA- wie auch UVB-Filter gibt es viele aus der Fachliteratur bekannte Substanzen, z.B. Benzylidenkampfer-Derivate wie 3-(4'-Methylbenzyliden)-di-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium-methylsulfat (z.B. Mexoryl® SK) oder (2-Oxoborn-3-yliden)toluol-4-sulfonsäure (z.B. Mexoryl® SL),

Benzoyl- oder Dibenzoylmethane wie 1-(4-tert-Butylphenyl)-3-(4-methoxyphenyl)propan-1,3-dion (z.B. Eusolex® 9020) oder 4-Isopropyldibenzoylmethan (z.B. Eusolex® 8020),

Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),

Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000),

Salicylatderivate wie 2-Ethylhexylsalicylat (z.B. Eusolex® OS), 4-lsopropylbenzylsalicylat (z.B. Megasol®) oder 3,3,5-Trimethylcyclohexylsalicylat (z.B. Eusolex® HMS),

4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25),

Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neo Heliopan® AP) oder 2,2-(1,4-Phenylen)-bisbenzimidazol-6-sulfonsäure;
und weitere Substanzen wie
- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]-hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin (z.B. Uvinul® T 150)
- 2-(4-Diethylamino-2-hydroxy-benzoyl)-benzoesäurehexylester (z.B. Uvinul® UVA Plus, Fa. BASF).

Die in der Liste aufgeführten Verbindungen sind nur als Beispiele aufzufassen. Selbstverständlich können auch andere UV-Filter verwendet werden.

Diese organischen UV-Filter werden in der Regel in einer Menge von 0,5 bis 10 Gewichtsprozent, vorzugsweise 1-8%, in kosmetische Formulierungen eingearbeitet.

Weitere geeignete organische UV-Filter sind z.B.
- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole^{®}),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb^{®} HEB),
- Dimethicondiethylbenzalmalonat (CAS-Nr. 207 574-74-1)
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1H-benzimidazol-4,6-disulfonsäure, Mononatriumsalz) (CAS-Nr. 180 898-37-7) und
- 2,4-Bis-{[4-(2-ethyl-hexyloxy)-2-hydroxyl]-phenyl}-6-(4-methoxyphenyl)-1,3,5-triazin (CAS-Nr. 103 597-45-, 187 393-00-6).

Weitere geeignete UV-Filter sind auch Methoxyflavone entsprechend der älteren deutschen Patentanmeldung DE 10232595.2.

Organische UV-Filter werden in der Regel in einer Menge von 0,5 bis 20 Gewichtsprozent, vorzugsweise 1-15%, in kosmetische Formulierungen eingearbeitet.

Als anorganische UV-Filter sind solche aus der Gruppe der Titandioxide wie z.B. gecoatetes Titandioxid (z.B. Eusolex® T-2000, Eusolex^{®}T-AQUA), Zinkoxide (z.B. Sachtotec®), Eisenoxide und auch Ceroxide denkbar.
Diese anorganischen UV-Filter werden in der Regel in einer Menge von 0,5 bis 20 Gewichtsprozent, vorzugsweise 2-10%, in kosmetische Zubereitungen eingearbeitet.

Bevorzugte Verbindungen mit UV-filternden Eigenschaften sind 3-(4'-Methylbenzyliden)-dl-kampfer, 1-(4-tert-Butylphenyl)-3-(4-methoxy-phenyl)-propan-1,3-dion, 4-Isopropyldibenzoylmethan, 2-Hydroxy-4-methoxybenzophenon, Methoxyzimtsäureoctylester, 3,3,5-Trimethyl-cyclohexylsalicylat, 4-(Dimethylamino)benzoesäure-2-ethylhexylester, 2-Cyano-3,3-diphenyl-acrylsäure-2-ethylhexylester, 2-Phenyl-benzimidazol-5-sulfonsäure sowie ihre Kalium-, Natrium- und Triethanolaminsalze.

Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten. Mit dieser Kombination ergibt sich ein Breitbandschutz, der durch Zusatz von anorganischen UV-Filtern, wie Titandioxid-Mikropartikeln noch ergänzt werden kann.

Alle genannten UV-Filter können auch in verkapselter Form eingesetzt werden. Insbesondere ist es von Vorteil, organische UV-Filter in verkapselter Form einzusetzen. Im Einzelnen ergeben sich die folgende Vorteile:
- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.
- Bestimmte UV-Filter, insbesondere Dibenzoylmethan-Derivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.
- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter oder anderer Inhaltsstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher ist es erfindungsgemäß bevorzugt, wenn ein oder mehrere der oben genannten UV-Filter in verkapselter Form vorliegen. Vorteilhaft ist es dabei, wenn die Kapseln so klein sind, dass sie mit dem bloßen Auge nicht zu sehen sind. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Erfindungsgemäß besonders bevorzugt einzusetzende Kapseln weisen Wände auf, die durch einen Sol-Gel-Prozess, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Siliciumoxid-hydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäßen Zubereitungen vorzugsweise in solchen Mengen enthalten, die gewährleisten, dass die verkapselten UV-Filter in den oben angegebenen Mengen in der Zubereitung vorliegen.

Die erfindungsgemäßen Zubereitungen können darüber hinaus weitere übliche hautschonende oder hautpflegende Wirkstoffe enthalten. Dies können prinzipiell alle den Fachmann bekannten Wirkstoffe sein.

Dies können Chromon-Derivate sein. Dabei werden vorzugsweise bestimmte Chromen-2-on-Derivate, die sich als Wirkstoffe zur vorbeugenden Behandlung von menschlicher Haut und menschlichem Haar gegen Alterungsprozesse und schädigende Umwelteinflüsse eignen, unter der Bezeichnung Chromon-Derivate verstanden. Sie zeigen gleichzeitig ein niedriges Irritationspotential für die Haut, beeinflussen die Wasserbindung in der Haut positiv, erhalten oder erhöhen die Elastizität der Haut und fördern somit eine Glättung der Haut. Diese Verbindungen entsprechen vorzugsweise der Formel II 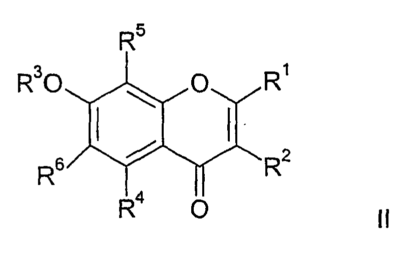 wobei
R¹ und R² gleich oder verschieden sein können und ausgewählt sind aus
- H, -C(=O)-R⁷, -C(=O)-OR⁷,
- geradkettigen oder verzweigten C₁- bis C₂₀-Alkylgruppen,
- geradkettigen oder verzweigten C₃- bis C₂₀-Alkenylgruppen,
- geradkettigen oder verzweigten C₁- bis C₂₀-Hydroxyalkylgruppen,
wobei die Hydroxygruppe an ein primäres oder sekundäres Kohlenstoffatom der Kette gebunden sein kann und weiter die Alkylkette auch durch Sauerstoff unterbrochen sein kann, und/oder
- C₃- bis C₁₀-Cycloalkylgruppen und/oder C₃- bis C₁₂-Cycloalkenylgruppen, wobei die Ringe jeweils auch durch -(CH₂)ₙ-Gruppen mit n = 1 bis 3 überbrückt sein können,
R³ für H oder geradkettige oder verzweigte C₁- bis C₂₀-Alkylgruppen steht, R⁴ für H oder OR⁸ steht,
R⁵ und R⁶ gleich oder verschieden sein können und ausgewählt sind aus
- -H, -OH,
- geradkettigen oder verzweigten C₁- bis C₂₀-Alkylgruppen,
- geradkettigen oder verzweigten C₃- bis C₂₀-Alkenylgruppen,
- geradkettigen oder verzweigten C₁- bis C₂₀-Hydroxyalkylgruppen,
wobei die Hydroxygruppe an ein primäres oder sekundäres Kohlenstoffatom der Kette gebunden sein kann und weiter die Alkylkette auch durch Sauerstoff unterbrochen sein kann und
R⁷ für H, geradkettige oder verzweigte C₁- bis C₂₀-Alkylgruppen, eine Polyhydroxy-Verbindung, wie vorzugsweise einen Ascorbinsäurerest oder glycosidische Reste steht und
R⁸ für H oder geradkettige oder verzweigte C₁- bis C₂₀-Alkylgruppen steht, wobei mindestens 2 der Substituenten R¹, R², R⁴-R⁶ verschieden von H sind oder mindestens ein Substituent aus R¹ und R² für -C(=O)-R⁷ oder -C(=O)-OR⁷ steht.

Der Anteil an einer oder mehreren aus Cromon-Derivaten ausgewählten Verbindungen in der erfindungsgemäßen Zubereitung beträgt vorzugsweise 0,001 bis 5 Gewichtsprozent, besonders bevorzugt 0,01 bis 2 Gewichtsprozent, bezogen auf die gesamte Zubereitung.

Eine Schutzwirkung der erfindungsgemäßen Formulierungen gegen oxidativen Stress oder gegen den Effekt freier Radikale lässt sich erzielen, wenn die Zubereitungen ein oder mehrere Antioxidantien enthalten, wobei es dem Fachmann keinerlei Schwierigkeiten bereitet, Antioxidantien auszuwählen, die in geeigneter Weise schnell oder protrahiert wirken.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-)Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakt, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-Acetat), Vitamin A und Derivate (z.B. Vitamin-A-Palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als Wirkstoffe Lecithin, L-(+)-Ascorbylpalmitat und Zitronensäure (z.B. Oxynex^{®} AP), natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004). Derartige Antioxidantien werden mit Verbindungen der Formel I in solchen Zusammensetzungen üblicherweise in Verhältnissen im Bereich von 1000:1 bis 1:1000, bevorzugt in Mengen von 100:1 bis 1:100 eingesetzt.

Die erfindungsgemäßen Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin (Vitamin B₆), Panthothensäure, Biotin, Folsäure und Cobalamin (Vitamin B₁₂) in den erfindungsgemäßen kosmetischen Zubereitungen enthalten, insbesondere bevorzugt Vitamin-A-Palmitat, Vitamin C und dessen Derivaten, DL-α-Tocopherol, Tocopherol-E-Acetat, Nicotinsäure, Pantothensäure und Biotin. Vitamine werden dabei mit Verbindungen der Formel I üblicherweise in Verhältnissen im Bereich von 1000:1 bis 1:1000, bevorzugt in Mengen von 100:1 bis 1:100 eingesetzt.

Unter den Phenolen mit antioxidativer Wirkung sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer zur Ketofunktion benachbarten OH-Gruppe oder OH-Gruppen in 3',4'- oder 6,7- oder 7,8-Position antioxidative Eigenschaften aufweisen, während einige andere Mono- und Dihydroxyflavone keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers, I.M.C.M. Rietjens; Free Rest Biology & Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete Antioxidantien sind weiter Verbindungen der Formel III 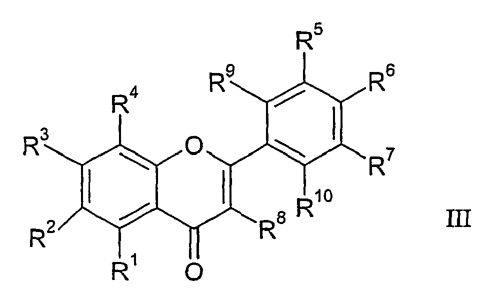 wobei R¹ bis R¹⁰ gleich oder verschieden sein können und ausgewählt sind aus
- H
- OR¹¹
- geradkettigen oder verzweigten C₁- bis C₂₀-Alkylgruppen,
- geradkettigen oder verzweigten C₃- bis C₂₀-Alkenylgruppen,
- geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- C₃- bis C₁₀-Cycloalkylgruppen und/oder C₃- bis C₁₂-Cycloalkenylgruppen, wobei die Ringe jeweils auch durch -(CH₂)ₙ-Gruppen mit n = 1 bis 3 überbrückt sein können,
- wobei alle OR¹¹ unabhängig voneinander stehen für
   - OH
   - geradkettige oder verzweigte C₁- bis C₂₀-Alkyloxygruppen,
   - geradkettigen oder verzweigten C₃- bis C₂₀-Alkenyloxygruppen,
   - geradkettigen oder verzweigten C₁- bis C₂₀-Hydroxyalkoxygruppen, wobei die Hydroxygruppe(n) an ein primäres oder sekundäres Kohlenstoffatom der Kette gebunden sein können und weiter die Alkylkette auch durch Sauerstoff unterbrochen sein kann, und/oder
   - C₃- bis C₁₀-Cycloalkyloxygruppen und/oder C₃- bis C₁₂-Cycloalkenyloxygruppen, wobei die Ringe jeweils auch durch -(CH₂)ₙ-Gruppen mit n = 1 bis 3 überbrückt sein können und/oder
   - Mono- und/oder Oligoglycosylreste,
      mit der Maßgabe, dass mindestens 4 Reste aus R¹ bis R⁷ für OH stehen und dass im Molekül mindestens 2 Paare benachbarter Gruppen -OH vorliegen,
- oder R², R⁵ und R⁶ für OH und die Reste R¹, R³, R⁴ und R⁷⁻¹⁰ für H stehen,
wie sie in der älteren deutschen Patentanmeldung DE 10244282 beschrieben sind.

Besonders bevorzugte Wirkstoffe sind Pyrimidincarbonsäuren und/oder Aryloxime.

Pyrimidincarbonsäuren kommen in halophilen Mikroorganismen vor und spielen bei der Osmoregulation dieser Organismen eine Rolle *(*E. A. Galinski et al., Eur. J. Biochem., 149 (1985) Seite 135-139*).* Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten, in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutisch wirksame Peptide und Proteine, z.B. t-PA, können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Grundierungen, Pflegecremes und Sonnenschutzpräparaten eingesetzt werden.

Dabei wird vorzugsweise eine Pyrimidincarbonsäure gemäß der unten stehenden Formel IV eingesetzt,

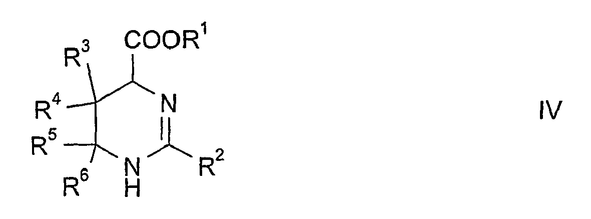

in der R¹ ein Rest H oder C₁₋₈-Alkyl, R² ein Rest H oder C₁₋₄-Alkyl und R³, R⁴, R⁵ sowie R⁶ jeweils unabhängig voneinander ein Rest aus der Gruppe H, OH, NH₂ und C₁₋₄-Alkyl sind. Bevorzugt werden Pyrimidincarbonsäuren eingesetzt, bei denen R² eine Methyl- oder eine Ethylgruppe ist und R¹ bzw. R⁵ und R⁶ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S,S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Dabei enthalten die Zubereitungen vorzugsweise 0,01 bis 10 Gew.-% des Aryloxims, wobei es insbesondere bevorzugt ist, wenn die Zubereitung 0,05 bis 5 Gew.-% Aryloxim enthält.

Ferner können die erfindungsgemäßen Zubereitungen auch Farbstoffe und Farbpigmente enthalten. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch.
Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| **Chemische oder sonstige Bezeichnung** | **CIN** | **Farbe** |
|---|---|---|
| Pigment Green | 10006 | grün |
| Acid Green 1 | 10020 | grün |
| 2,4-Dinitrohydroxynaphthalin-7-sulfonsäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfonsäurediethylamid-1'-phenylazo)- | 12490 | rot |
| 3-hydroxy-5"-chlor-2",4"-dimethoxy-2- | | |
| naphthoesäureanilid | | |
| Disperse Yellow 16 | 12700 | gelb |
| 1-(4-Sulfo-1-phenylazo)-4-amino-benzol-5-sulfosäure | 13015 | gelb |
| 2,4-Dihydroxy-azobenzol-4'-sulfosäure | 14270 | orange |
| 2-(2,4-Dimethylphenylazo-5-sulfosäure)-1- | 14700 | rot |
| hydroxynaphthalin-4-sulfosäure | | |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2- | 15525 | rot |
| hydroxynaphthalin | | |
| 1-(3-Methyl-phenylazo-4-sulfosäure)-2- | 15580 | rot |
| hydroxynaphthalin | | |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2- | 15850 | rot |
| naphthylcarbonsäure | | |
| 1-(2-Sulfo-4-methyl-5-chlor-1-phenylazo)-2-hydroxy- | 15865 | rot |
| naphthalin-3-carbonsäure | | |
| 1-(2-Sulfo-1-naphthylazo)-2-hydroxynaphthalin-3- | 15880 | rot |
| carbonsäure | | |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8-trisulfosäure | 16290 | rot |
| 8-Amino-2-phenylazo-1-naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy- | 19140 | gelb |
| pyrazolon-3-carbonsäure | | |
| Pigment Yellow 16 | 20040 | gelb |
| 2,6-(4'-Sulfo-2",4"-dimethyl)-bis-phenylazo)-1,3- | 20170 | orange |
| dihydroxybenzol | | |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1"-phenylazo)-7'-sulfo-1'-naphthylazo]-1- | 27755 | schwarz |
| hydroxy-7-aminonaphthalin-3,6-disulfosäure | | |
| 4-[4"-Sulfo-1"-phenylazo)-7'-sulfo-1'-naphthylazo]-1- | 28440 | schwarz |
| hydroxy-8-acetyl-aminonaphthalin-3,5-disulfosäure | | |
| Direct Orange 34, 39, 44, 46, 60 | 40215 | orange |
| Food Yellow | 40800 | orange |
| trans-β-Apo-8'-carotinaldehyd (C₃₀) | 40820 | orange |
| trans-Apo-8'-carotinsäure (C₃₀)-ethylester | 40850 | orange |
| Canthaxanthin | 40850 | orange |
| Acid Blue 1 | 42045 | blau |
| 2,4-Disulfo-5-hydroxy-4'-4"-bis-(diethylamino)triphenyl- | 42051 | blau |
| carbinol | | |
| 4-[(-4-N-Ethyl-p-sulfobenzylamino)-phenyl-(4-hydroxy-2- | 42053 | grün |
| sulfophenyl)-(methylen)-1-(N-ethyl-N-p-sulfobenzyl)-2,5- | | |
| cyclohexadienimin] | | |
| Acid Blue 7 | 42080 | blau |
| (N-Ethyl-p-sulfobenzyl-amino)-phenyl-(2-sulfophenyl)- | 42090 | blau |
| methylen-(N-ethyl-N-p-sulfo-benzyl) Δ^{2,5}- | | |
| cyclohexadienimin | | |
| Acid Green 9 | 42100 | grün |
| Diethyl-di-sulfobenzyl-di-4-amino-2-chlor-di-2-methyl- | 42170 | grün |
| fuchsonimmonium | | |
| Basic Violet 14 | 42510 | violett |
| Basic Violet 2 | 42520 | violett |
| 2'-Methyl-4'-(N-ethyl-N-m-sulfobenzyl)-amino-4"-(N- | 42735 | blau |
| diethyl)-amino-2-methyl-N-ethyl-N-m-sulfobenzyl- | | |
| fuchsonimmonium | | |
| 4'-(N-Dimethyl)-amino-4"-(N-phenyl)-aminonaphtho-N- | 44045 | blau |
| dimethylfuchsonimmonium | | |
| 2-Hydroxy-3,6-disulfo-4,4'-bis-dimethylaminonaphtho- | 44090 | grün |
| fuchsonimmonium | | |
| Acid Red 52 | 45100 | rot |
| 3-(2'-Methylphenylamino)-6-(2'-methyl-4'- | 45190 | violett |
| sulfophenylamino)-9-(2"-carboxyphenyl)- | | |
| xantheniumsalz | | |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidino)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanin | 74160 | blau |
| Direct Blue 86 | 74180 | blau |
| Chloriertes Phthalocyanin | 74260 | grün |
| Natural Yellow 6, 19; Natural Red 1 | 75100 | gelb |
| Bixin, Nor-Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)-1,6-heptadien- | 75300 | gelb |
| 3,5-dion | | |
| Komplexsalz (Na, Al, Ca) der Karminsäure | 75470 | rot |
| Chlorophyll a und b; Kupferverbindungen der | 75810 | grün |
| Chlorophylle und Chlorophylline | | |
| Aluminium | 77000 | weiß |
| Tonerdehydrat | 77002 | weiß |
| Wasserhaltiges Aluminiumsilikat | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268 | schwarz |
| Chromoxid | 77288 | grün |
| Chromoxid, wasserhaltig | 77278 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)- | 77510 | blau |
| hexacyanoferrat | | |
| Pigment White 18 | 77713 | weiß |
| Manganammoniumdiphosphat | 77742 | violett |
| Manganphosphat; Mn₃(PO₄)₂· 7 H₂O | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalze, Anthocyane | | rot |
| Aluminium-, Zink-, Magnesium- und Calciumstearat | | weiß |
| Bromthymolblau | | blau |

Es kann ferner günstig sein, als Farbstoff eine oder mehrere Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfonsäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfonsäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77492), Manganammoniumdiphosphat und Titandioxid.

Ferner vorteilhaft sind öllösliche Naturfarbstoffe, wie z. B. Paprikaextrakt, β-Carotin oder Cochenille.

Vorteilhaft im Sinne der vorliegenden Erfindung sind ferner Gelcremes mit einem Gehalt an Perlglanzpigmenten. Bevorzugt sind insbesondere die im folgenden aufgelisteten Arten von Perlglanzpigmenten:
1. natürliche Perlglanzpigmente, wie z. B.
   1. "Fischsilber" (Guanin/Hypoxanthin-Mischkristalle aus Fischschuppen) und
   2. "Perlmutt" (vermahlene Muschelschalen)
2. monokristalline Perlglanzpigmente wie z. B. Bismutoxychlorid (BiOCl)
3. Schicht-Substrat-Pigmente: z. B. Glimmer/Metalloxid.

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Besonders vorteilhaft ist z.B. das unter der CIN 77163 aufgelistete Glanzpigment.

Vorteilhaft sind ferner beispielsweise die folgenden Perlglanzpigmentarten auf Basis von Glimmer/Metalloxid:

| **Gruppe** | **Belegung/Schichtdicke** | **Farbe** |
|---|---|---|
| **Silberweiße** | TiO₂: 40-60 nm | silber |
| **Perlglanzpigmente** | | |
| **Interferenzpigmente** | TiO₂: 60-80 nm | gelb |
| | TiO₂: 80-100 nm | rot |
| | TiO₂: 100-140 nm | blau |
| | TiO₂: 120-160 nm | grün |
| **Farbglanzpigmente** | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| **Kombinationspigmente** | TiO₂ / Fe₂O₃ | Goldtöne |
| | TiO₂ / Cr₂O₃ | grün |
| | TiO₂ / Berliner Blau | tiefblau |

Besonders bevorzugt sind z. B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Perlglanzpigmente, welche unter Verwendung von SiO₂ hergestellt werden. Solche Pigmente, die auch zusätzlich goniochromatische Effekte haben können, sind z.B. unter dem Handelsnamen Sicopearl Fantastico bei der Firma BASF erhältlich.

Weiterhin vorteilhaft können Pigmente der Firma Engelhard/Mearl auf Basis von Calcium-Natrium-Borosilikat, die mit Titandioxid beschichtet sind, eingesetzt werden. Diese sind unter dem Namen Reflecks erhältlich. Sie weisen durch ihre Partikelgröße von 40-80 µm zusätzlich zur Farbe einen Glitzereffekt auf.

Besonders vorteilhaft sind ferner auch Effektpigmente, welche unter der Handelsbezeichnung Metasomes Standard/Glitter in verschiedenen Farben (gelb, rot, grün, blau) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (Cl) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z.B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Alle Verbindungen oder Komponenten, die in den Zubereitungen verwendet werden können, sind entweder bekannt und käuflich erwerbbar oder können nach bekannten Verfahren synthetisiert werden.

Die erfindungsgemäßen Zubereitungen können außerdem weitere übliche hautfreundliche oder hautpflegende Wirkstoffe enthalten. Hierbei kann es sich im Prinzip um alle dem Fachmann bekannten Wirkstoffe handeln.

Die Zusammensetzung der vorliegenden Erfindung kann in Form von flüssigen, cremigen, milchigen oder gelartigen Badezusätzen, die zusammen mit dem Badewasser als Flüssigkeit zugegeben werden, oder in Badekapseln, die vorzugsweise aus Gelatine bestehen und sich im Badewasser auflösen und die Zusammensetzung der vorliegenden Erfindung freigeben, vorliegen.

Die vorliegende Erfindung stellt daher außerdem eine mindestens eine selbstbräunende Substanz enthaltende Zusammensetzung bereit, die dadurch gekennzeichnet ist, dass die Formulierung ein flüssiger, cremiger, milchiger und/oder gelartiger Badezusatz, eine Badetablette, ein Badesalz und/oder eine Badekapsel ist.

Eine mögliche Zusammensetzung der Flüssigformulierung enthält bis zu 75% Tenside (anionisch, kationisch, nonionisch oder amphoterisch), bis zu 10% Viskositätsmittel wie Fettalkohole, bis zu 5% Kompatibilitäts- und Konditionierungsmittel, bis zu 5% weitere Inhaltsstoffe, wie Rückfettungsmittel, Verdickungsmittel, Trübungsmittel oder Pigmente, bis zu 5% Parfümöle, bis zu 1 % Konservierungsmittel, bis zu 0,5% Komplexbildner, bis zu 1% Farbstoffe, 0,1-1% DHA, UV-Filter, 0,1-20% Propylenglykol und/oder Glycerol und 0,1 bis 10% fettige Träger, Rest zu 100% Wasser.

Die Zusammensetzung der vorliegenden Erfindung kann auch in Badezusätzen wie Badetabletten oder -salzen vorliegen. Eine mögliche Zusammensetzung der festen Formulierung enthält bis zu 90% Natriumsalze (z.B. Natriumcarbonat, -bicarbonat, -sesquicarbonat, -chlorid, -thiosulfat, -borat, -phosphat oder -citrat), bis zu 40% organische Säuren (z.B. Weinsäure, Zitronensäure) für schäumende Zubereitungen, bis zu 5% Parfümöle (essentielle Öle), bis zu 5% hautpflegende Substanzen, bis zu 5% Pflanzenöle, bis zu 5% Füllstoffe und für Tabletten Zerfallhilfsmittel (z.B. Dextrin, Silica, Cellulose, Gummi), bis zu 5% Bindemittel, bis zu 2% Tenside, bis zu 1% Farbstoffe, 0,1-1% DHA, UV-Filter, 0,1-20% Propylenglykol und/oder Glycerol und 0,1 bis 10% fettige Träger.

Weiterhin ist es bevorzugt, wenn die selbstbräunenden Formulierungen feuchtigkeitsspendende Substanzen wie beispielsweise Erythrulose oder die oben genannten Ectoine enthalten.

Überraschenderweise wurde nun gefunden, dass die erforderliche Wirkstoffkonzentration der Verbindungen gemäß Formel I und der zusätzlichen Bräunungsmittel reduziert werden kann, wenn die selbstbräunenden Mittel bei erhöhter Temperatur angewendet werden.

Man nimmt an, dass der verbesserte Bräunungseffekt bei erhöhten Temperaturen bei 1,3-Dihydroxyaceton mit dem folgenden Mechanismus im Zusammenhang steht. Als Rohmaterial liegt DHA in Form eines Pulvers vor und besteht aus Dimeren. In Wasser gelöst wandeln sich einige der Dimere in die aktive monomere Form um, was die Bräunungsreaktion herbeiführt. Bei erhöhter Wassertemperatur nimmt die Menge an Monomeren zu. Es zeigte sich beispielsweise, dass in DHA-Lösungen bei 30-50°C bis zu 30% mehr aktive DHA-Monomere vorliegen als in DHA-Lösungen bei 20°C. Gleichzeitig steigert die erhöhte Temperatur die Reaktionsgeschwindigkeit der Bräunungsreaktion.

Die vorliegende Erfindung stellt daher die Verwendung mindestens einer selbstbräunenden Substanz gemäß Formel I oder einer Formulierung enthaltend mindestens eine selbstbräunende Substanz gemäß Formel I für die Anwendung auf der menschlichen Haut bereit, wobei die Anwendung bei erhöhter Temperatur stattfindet.

Aus diesem Grunde ist es erfindungsgemäß bevorzugt, wenn die Anwendungstemperatur im Bereich zwischen 25 und 60°C, vorzugsweise zwischen 30 und 55°C und besonders bevorzugt zwischen 37 und 50°C liegt.

Die vorliegende Erfindung stellt weiterhin ein Verfahren zum Bräunen des menschlichen Körpers bereit, das dadurch gekennzeichnet ist, dass man mindestens eine selbstbräunende Substanz gemäß Formel I oder eine Formulierung enthaltend mindestens eine selbstbräunende Substanz gemäß Formel I in Wasser löst, die Lösung auf eine Temperatur bringt, die gegenüber der Raumtemperatur erhöht ist, und die Lösung auf den menschlichen Körper aufträgt.

Weiter wurde gefunden, dass sich das Gleichgewicht zwischen Monomer- und Dimerkonzentration innerhalb etwa 15 Minuten nach Lösung einstellt. Es ist daher erfindungsgemäß bevorzugt, wenn man die Lösung der selbstbräunenden Substanz etwa 15 min, mindestens aber etwa 10 min erhitzt, bevor die Lösung auf die menschliche Haut aufgetragen wird.

Auf besonders bequeme Weise kann der genannte Effekt bei der Verwendung in Badewannen genutzt werden.

Die erforderliche gleichmäßige Bräunung lässt sich durch bloßes Reiben oft nur mit Schwierigkeiten, wenn überhaupt erzielen. Außerdem sind einige Bereiche des Körpers, insbesondere auf dem Rücken, beim Selbstauftrag einer Creme nur mit Schwierigkeiten zu erreichen. Diese Probleme werden bei einer Anwendung als Badewasser vermieden. Außerdem kann die Anwendung während der normalen Badezeit stattfinden und die Penetration der selbstbräunenden Mittel in die tieferen Schichten verhornter Haut wird durch das Aufweichen der Haut beim Baden gefördert.

Es ist erfindungsgemäß daher besonders bevorzugt, wenn die Lösung in einer Badewanne oder einem Whirlpool angewendet wird. Der intensive und lange andauernde Kontakt der Haut mit der Wirkstofflösung sorgt außerdem für eine besonders gleichmäßige Bräunung, was überdies mit besonders niedrigen Wirkstoffkonzentrationen möglich ist.

Whirlpools oder andere Bäder mit bewegter Oberfläche bieten insbesondere den zusätzlichen Vorteil, dass im Halsbereich keine Linie entsteht, sondern die Bräune allmählich schwächer wird. Wenn das Gesicht ebenfalls gebräunt werden soll, so kann dies auf herkömmliche Weise durch Anwendung einer Selbstbräuner enthaltenden Creme oder durch Einsprühen mit einer Selbstbräuner-Lösung erfolgen.

Im entsprechenden erfindungsgemäßen Verfahren wird der menschliche Körper ganz oder teilweise in die Lösung eingetaucht.

Ein weiteres erfindungsgemäß bevorzugtes Verfahren zum Auftragen von Selbstbräuner-Lösungen auf die Haut ist das Einsprühen, das beispielsweise mit einer Dusche oder Spritzpistole erfolgen kann.

Im entsprechenden Verfahren zur gleichmäßigen Bräunung wird der menschliche Körper gleichmäßig ganz oder teilweise mit der erhitzten Lösung eingesprüht.

Die auf diese Weise erziele Bräunung der Haut lässt sich nicht abwaschen und verschwindet erst mit der normalen Schuppung der Haut (nach etwa 10-15 Tagen).

Insbesondere bei der Anwendung als Bad kann es weiter von Vorzug sein, wenn auf Körperpartien, die nicht oder nur wenig gebräunt werden sollen, eine wasserabstoßende Zubereitung aufgetragen wird. Solche Zubereitungen können auf der Grundlage von Silikonen, Paraffinen, verschiedenen organischen Polymeren, Vaseline oder Fettsäuresalzen, wie Stearaten, hergestellt werden. Beim Baden verhindern oder verringern sie den Kontakt der behandelten Haut mit dem selbstbräunenden Mittel und so die erzielte Bräune. Besonders bei Körperpartien mit verdickter verhornter Haut kann eine solche Vorbehandlung ratsam sein, um eine intensive Färbung dieser Bereiche zu verhindern.

Eine andere Aufgabe der Erfindung betrifft die Verwendung dieser kosmetischen oder dermatologischen Zusammensetzungen für die künstliche Färbung der Haut.

Eine weitere Aufgabe der Erfindung betrifft ein Verfahren zur künstlichen Färbung der Haut unter Verwendung, durch Auftragen, einer oben im Text beschriebenen Zusammensetzung.

Nachdem die Erfindung nun allgemein beschrieben wurde, ergibt sich aus der Bezugnahme auf einige spezifische Beispiele, die hier ausschließlich zur Veranschaulichung dienen und, soweit nicht anders angegeben, nicht als einschränkend zu verstehen sind, ein genaueres Verständnis.

Im Folgenden werden nicht einschränkende Beispiele erfindungsgemäßer Zusammensetzungen gegeben.

Auch wenn die vorliegende Erfindung unter Bezugnahme auf spezifische Details bestimmter Ausführungsformen der Erfindung beschrieben wurde, ist es nicht beabsichtigt, dass solche Details als Einschränkungen des Umfanges der Erfindung zu betrachten sind, außer wenn diese in den beiliegenden Ansprüchen enthalten sind.

### Beispiele

### Beispiel 1: Synthese des 2,5-Dipalmitoyloxymethyl-[1,4]-dioxan-2,5-diol

### Verfahren A

Eine Lösung von DHA (40,0 g, 0,44 mol) in Dichlormethan (330 ml) und Pyridin (130 ml) wird bei 0°C unter einer Stickstoffatmosphäre mit Palmitoylchlorid (14,4 ml, 0,05 mol) versetzt und bei Raumtemperatur 24 h gerührt. Die Reaktion wird durch langsame Zugabe einer wässrigen Lösung von 1 N HCl abgeschreckt und das entstandene Gemisch zwischen 1 N HCl (250 ml) und Dichlormethan (250 ml) verteilt. Die organische Phase wird mit gesättigtem Natriumbicarbonat gewaschen, über MgSO₄ getrocknet und im Vakuum eingeengt. Der entstandene feste Rückstand wird vorsichtig aus Toluol umkristallisiert; man erhält 8,3 g des 2,5-Dipalmitoyloxymethyl-[1,4]-dioxan-2,5-diols als weiße Nadeln. Die Monomerform, die im Filtrat vorliegt, wandelt sich bei Stehenlassen bei Raumtemperatur langsam wieder in das Dimer 2,5-Dipalmitoyloxymethyl-[1,4]-dioxan-2,5-diol um.

### Verfahren B

In einem 500 ml fassenden 2-Hals-Rundkolben werden bei Raumtemperatur unter einer Stickstoffatmosphäre Palmitinsäure (5,70 g, 22,2 mmol) und DMAP (2,71 g, 22,2 mmol) zu einer gerührten Lösung von DHA (1,00 g, 11,1 mmol) in CH₂Cl₂ (200 ml) gegeben. Eine Lösung von DCC (4,59 g, 22,2 mmol) in CH₂Cl₂ (20 ml) wird zugetropft. Nach 20-stündigem Rühren bei Raumtemperatur wird der ausgefallene Dicyclohexylharnstoff abfiltriert. Der Dicyclohexylharnstoff wird mit einem Überschuss an CH₂Cl₂ gewaschen, um eventuell verbliebenes Produkt zu entfernen. Das Filtrat wird dann mit einer 5%igen wässrigen HCl-Lösung (100 ml) und danach mit destilliertem H₂O (200 ml) und gesättigter NaCl-Lösung (150 ml) gewaschen. Die organische Schicht wird dann mit Natriumsulfat getrocknet und unter verringertem Druck eingedampft. Nach dem Eindampfen wird der Rückstand aus EtOAc/Petrolether umkristallisiert; man erhält farblose Kristalle des 2,5-Dipalmitoyloxymethyl-[1,4]-dioxan-2,5-diols.
Smp.: 95,2°C
¹H NMR (500 MHz, CDCl₃) δ: 4,23 (d, 2H, J=11,8 Hz), 4,08 (d, 2H, J=11,8 Hz), 4,00 (d, 2H, J=11,8 Hz), 3,60 (d, 2H, *J*=11,8 Hz), 3,10 (s, 2H), 2,37 (t, 4H, J=7,5 Hz), 1,60-1,66 (m, 4H), 1,26-1,29 (m, 48H), 0,88 (t, 6H, J=6,8 Hz).
HPLC-APCI-MS: 674 (M⁺+NH₄⁺)

Durch Verwendung analoger Reaktionen sind analoge dimere DHA-Ester mit unterschiedlichen Alkylkettenlängen, wie 1,3-Didodecansäure-2-oxopropylester oder 1,3-ditetradecansäure-2-oxopropylester erhältlich.

### Beispiel 2: Schaumbad

| **Inhaltsstoff** | **[%]** |
|---|---|
| 2,5-Dipalmitoyloxymethyl- | 0,1-1 |
| [1,4]-dioxan-2,5-diol | |
| Tensid | 10-20 |
| Phospholipide | 5 |
| Konservierungsmittel | q.s. |
| Färbemittel | q.s. |
| Parfümöl | q.s. |
| Wasser | ad 100 |

Herstellung:
Die Inhaltsstoffe werden vermischt.

### Beispiel 3: Formulierung zum Versprühen in Wirkstoffduschen

| **Inhaltsstoff** | **[%]** |
|---|---|
| 2,5-Dipalmitoyloxymethyl- | 5 |
| [1,4]-dioxan-2,5-diol | |
| Propylenglykol | 10 |
| Phospholipide | 5 |
| Konservierungsmittel | q.s. |
| Parfümöl | q.s. |
| Wasser | ad 100 |

Herstellung:
Die Inhaltsstoffe werden vermischt.

### Beispiel 4: Selbstbräunende Sonnencreme

### A. Emulsion enthaltend den Dihydroxyacetonester 2,5-Dipalmitoyloxymethyl-[1,4]-dioxan-2,5-diol:

| Ölphase: | |
|---|---|
| Steareth-2 (Tensid) | 3% |
| Steareth-21 (Tensid) | 2% |
| PPG-15-stearylether (Tensid) | 29,5% |
| 2,5-Dipalmitoyloxymethyl-[1,4]-dioxan-2,5-diol | 14,6% |

| Wasserphase: | |
|---|---|
| Phenoxyethanol (Konservierungsmittel) | 0,5% |
| Wasser | qs 100% |

### B. Lipasehaltige Emulsion:

| Ölphase: | |
|---|---|
| Steareth-2 (Tensid) | 3% |
| Steareth-21 (Tensid) | 2% |
| PPG-15-stearylether (Tensid) | 29,5% |

| Wasserphase: | |
|---|---|
| Phenoxyethanol (Konservierungsmittel) | 0,5% |
| Lipase SP644 | 2% |
| Wasser | qs 100% |

Die Emulsionen A und B werden in zwei getrennte Fächer eingebracht und zum Zeitpunkt des Auftragens auf die Haut miteinander vermischt.

Nach dem Auftragen auf die Haut verleiht das erhaltene Produkt der Haut eine zunehmend sonnengebräunte Färbung.

### Beispiel 5: Selbstbräunende Sonnencreme

### A. Emulsion enthaltend 2,5-Dipalmitoyloxymethyl-[1,4]-dioxan-2,5-diol:

| Ölphase: | |
|---|---|
| Steareth-2 (Tensid) | 3% |
| Steareth-21 (Tensid) | 2% |
| PPG-15-stearylether (Tensid) | 29,5% |
| 2,5-Dipalmitoyloxymethyl-[1,4]-dioxan-2,5-diol | 10% |

| Wasserphase: | |
|---|---|
| Phenoxyethanol (Konservierungsmittel) | 0,5% |
| Wasser | qs 100% |

### B. Lipasehaltige Emulsion:

| Ölphase: | |
|---|---|
| Steareth-2 (Tensid) | 3% |
| Steareth-21 (Tensid) | 2% |
| PPG-15-stearylether (Tensid) | 29,5% |

| Wasserphase: | |
|---|---|
| Phenoxyethanol (Konservierungsmittel) | 0,5% |
| Lipase 100 L | 1 % |
| Wasser | qs 100% |

Die Emulsionen werden in zwei unterschiedliche Fächer eingebracht und zum Zeitpunkt der Anwendung in Kontakt gebracht.

### Beispiel 6: Selbstbräunende Sonnencreme

### A. Emulsion enthaltend 2,5-Dipalmitoyloxymethyl-[1,4]-dioxan-2,5-diol:

| Ölphase: | |
|---|---|
| Steareth-2 (Tensid) | 3% |
| Steareth-21 (Tensid) | 2% |
| PPG-15-stearylether (Tensid) | 29,5% |
| 2,5-Dipalmitoyloxymethyl-[1,4]-dioxan-2,5-diol | 10% |

| Wasserphase: | |
|---|---|
| Phenoxyethanol (Konservierungsmittel) | 0,5% |
| Wasser | qs 100% |

### B. Lysinhaltige Emulsion:

| Ölphase: | |
|---|---|
| Steareth-2 (Tensid) | 3% |
| Steareth-21 (Tensid) | 2% |
| PPG-15-stearylether (Tensid) | 29,5% |

| Wasserphase: | |
|---|---|
| Phenoxyethanol (Konservierungsmittel) | 0,5% |
| Lysin | 5% |
| Wasser | qs 100% |

Die Emulsionen A und B werden in zwei getrennte Fächer eingebracht und zum Zeitpunkt des Auftragens auf die Haut miteinander vermischt.

Nach dem Auftragen auf die Haut verleiht das erhaltene Produkt der Haut eine zunehmend sonnengebräunte Färbung.

### Beispiel 7: Maillard-Bräunungstest

Das Vermögen von Verbindungen gemäß Formel I, eine künstliche Bräunung der Haut herbeizuführen, lässt sich mit einem einfachen Maillard-Bräunungstest grob abschätzen. Dieser visuelle Test beruht auf dem Prinzip, dass die Reaktion der Haut mit DHA zur Erzeugung einer künstlichen Bräunung über ein komplexes Reaktionsgefüge (Maillard-Reaktion) mit freien Aminosäuren der Hautproteine, insbesondere mit Arginin, Lysin und Histidin, verläuft.

Das Bräunungsvermögen von 2,5-Dipalmitoyloxymethyl-[1,4]-dioxan-2,5-diol (A) wird über den Maillard-Bräunungseffekt nach Reaktion mit Lysin in verschiedenen Lösungsmitteln (Wasser und das kosmetische Öl Miglyol) beurteilt und mit denen von Referenzverbindungen verglichen: DHA und monomerer DHA-palmitinsäuremonoester (B) unter den gleichen Bedingungen. Daneben wird auch der Bräunungseffekt des 2,5-Dipalmitoyloxymethyl-[1,4]-dioxan-2,5-diols in Gegenwart von Lipasen (Enzymen, die der Körper zur Fetthydrolyse verwendet) untersucht.

| **Zeit** | **A** | **B** | **DHA** |
|---|---|---|---|
| 20 min | - | - | - |
| 1 h | - | Hellgelb | - |
| 5 h | Hellgelb | Gelb | Hellgelb |
| 1 Tag | Gelb | Braun | Braun |
| 3 Tage | " | " | " |
| 8 Tage | Braun | Dunkelbraun | Dunkelbraun |

**Bräunungstest 1:** 1 ml einer 0,1 M Lösung oder Suspension der Testsubstanz in Miglyol wird bei Raumtemperatur mit 1 ml einer 1 M wässrigen Lysinlösung reagieren gelassen.

| **Zeit** | **A** | **A+1% Lipase** | **B** | **B+1% Lipase** | **DHA** |
|---|---|---|---|---|---|
| 20 min | - | - | - | - | Hellgelb |
| 1 h | - | - | Hellgelb | Gelb | Braun |
| 5 h | - | Hellgelb | Gelb | Dunkelgelb | Dunkelbraun |
| 1 Tag | Hellgelb | Dunkelgelb | Braun | Braun | " |
| 2 Tage | Braun | Dunkelbraun | Dunkelbraun | Dunkelbraun | " |

**Bräunungstest 2:** 1 ml einer 0,1M **wässrigen Lösung** oder Suspension der Testsubstanz wird bei Raumtemperatur mit 1 ml einer 1 M wässrigen Lysinlösung reagieren gelassen.

Die Ergebnisse zeigen, dass die Verbindung A in der Lage ist, an Maillard-Bräunungsreaktionen teilzunehmen. Die Gegenwart von Lipasen fördert das Erscheinen der künstlichen Bräunung.

## Patentansprüche

1. Kosmetische oder dermatologische Zusammensetzung, die in einem kosmetisch oder dermatologisch verträglichen Träger mindestens ein verestertes Dihydroxyaceton-Derivat mit der allgemeinen Formel I 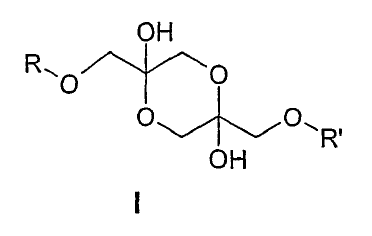 enthält, worin R und R' ein Wasserstoffatom oder einen gesättigten oder ungesättigten, linearen, verzweigten oder cyclischen, gegebenenfalls hydroxylierten Acylrest mit 2 bis 25 Kohlenstoffatomen bedeuten, wobei R und R' gleich oder verschieden sein können unter der Bedingung, dass sie nie gleichzeitig ein Wasserstoffatom bedeuten.

2. Zusammensetzung nach Anspruch 1, weiterhin enthaltend mindestens eine zur Spaltung mindestens einer Esterbindung befähigte Verbindung.

3. Zusammensetzung nach Anspruch 1 oder 2, in der der Acylrest ein Benzoylrest, ein Alkylbenzoylrest, ein Acylbenzoylrest oder ein 2-Hydroxy-2-phenylacetylrest ist.

4. Zusammensetzung nach mindestens einem der vorhergehenden Ansprüche, in der der Acylrest hydroxyliert ist.

5. Zusammensetzung nach mindestens einem der vorhergehenden Ansprüche, in der der Acylrest 3 bis 18 Kohlenstoffatome hat und in der das veresterte Derivat vorzugsweise aus der Gruppe 1,3-Didodecansäure-2-oxopropylester, 1,3-Dihexadecansäure-2-oxopropylester und 3-Hexadecansäure-2-oxopropylester ausgewählt ist.

6. Zusammensetzung nach mindestens einem der vorhergehenden Ansprüche, in der das veresterte Derivat in einer Konzentration im Bereich von 0,1 Gew.-% bis 20 Gew.-%, vorzugsweise von 0,5 Gew.-% bis 10 Gew.-%, bezogen auf das Gesamtgewicht der Zusammensetzung, vorliegt.

7. Zusammensetzung nach Anspruch 2, in der die zur Spaltung mindestens einer Esterbindung befähigte Verbindung eine nukleophile Verbindung, vorzugsweise ein Amin, ein Anion oder ein Enzym, besonders bevorzugt ein hydroxyliertes Amin, ein Carboxylatanion oder eine Hydrolase ist.

8. Zusammensetzung nach Anspruch 2 oder 7, in der die zur Spaltung mindestens einer Esterbindung befähigte Verbindung ausgewählt ist aus der Gruppe 3-Amino-1,2-propandiol, 2-Amino-2-methyl-1,3-propandiol, 2-Amino-2-methylpropanol, 2-Amino-2-hydroxymethyl-1,3-propandiol, Glucamin oder N-Methylglucamin, ein Lysin, Arginin und ein Histidin.

9. Zusammensetzung nach Anspruch 2, in der die zur Spaltung mindestens einer Esterbindung befähigte Verbindung eine Lipase, eine Esterase oder eine Protease, vorzugsweise eine Lipase ist.

10. Zusammensetzung nach mindestens einem der Ansprüche 2 oder 7 bis 9, in der die zur Spaltung mindestens einer Esterbindung befähigte Verbindung in einer Konzentration im Bereich von 0,1 Gew.-% bis 30 Gew.-%, vorzugsweise 0,5 Gew.-% bis 15 Gew.-%, bezogen auf das Gesamtgewicht der Zusammensetzung, vorliegt.

11. Zusammensetzung nach mindestens einem der Ansprüche 2 oder 7 bis 10, in der das veresterte Derivat und die zur Spaltung mindestens einer Esterbindung befähigte Verbindung so verpackt sind, dass sie nicht miteinander in Kontakt stehen.

12. Zusammensetzung nach Anspruch 11, in der das veresterte Derivat und die zur Spaltung mindestens einer Esterbindung befähigte Verbindung in einer einzigen Verpackung mit zwei Fächern enthalten sind.

13. Zusammensetzung nach Anspruch 12, in der mindestens eines des veresterten Derivats und der zur Spaltung mindestens einer Esterbindung befähigten Verbindung verkapselt ist.

14. Zusammensetzung nach mindestens einem der vorhergehenden Ansprüche, **dadurch gekennzeichnet, dass** die Zusammensetzung mindestens einen fettigen Träger und mindestens ein hydrophiles Lösungsmittel enthält, wobei der anwesende fettige Träger vorzugsweise aus einer oder mehreren Verbindungen der Ceramide, Cholesterin, Phospholipide, Cholesterylsulfat, Cholesterylphosphat, Phosphatidylcholin, Lecithin und/oder leeren Liposome ausgewählt ist und der fettige Träger vorzugsweise in einer Konzentration von 0,1 bis 10 Gew.-% vorliegt.

15. Zusammensetzung nach mindestens einem der vorhergehenden Ansprüche, **dadurch gekennzeichnet, dass** die Formulierung einen oder mehrere UV-Filter enthält.

16. Zusammensetzung nach Anspruch 14, **dadurch gekennzeichnet, dass** die Formulierung als hydrophiles Lösungsmittel Glycerol und/oder Propylenglykol, vorzugsweise in einer Konzentration von 0,1 to 20 Gew.-% enthält.

17. Zusammensetzung nach mindestens einem der vorhergehenden Ansprüche, **dadurch gekennzeichnet, dass** die Formulierung ein flüssiger, cremiger, milchiger und/oder gelartiger Badezusatz, eine Badetablette, ein Badesalz und/oder eine Badekapsel ist.

18. Verfahren zum Färben der Haut, das darin besteht, dass man darauf eine Zusammensetzung nach mindestens einem der Ansprüche 1 bis 17 aufträgt.

19. Verfahren zum Färben der Haut nach Anspruch 18 mit Anwendung bei erhöhter Temperatur, worin die Anwendungstemperatur vorzugsweise im Bereich zwischen 25 und 60°C, mehr bevorzugt zwischen 30 und 55°C und besonders bevorzugt zwischen 37 und 50°C liegt.

20. Verfahren zum Färben der Haut nach mindestens einem der Ansprüche 18 oder 19, **dadurch gekennzeichnet, dass** die Anwendung in einer Badewanne oder einem Whirlpool stattfindet.

21. Verfahren zum Färben der Haut nach mindestens einem der Ansprüche 18 oder 19, **dadurch gekennzeichnet, dass** das Auftragen auf die Haut durch Einsprühen, vorzugsweise mit einer Dusche oder einer Spritzpistole stattfindet.

22. Verfahren zum Bräunen des menschlichen Körpers, **dadurch gekennzeichnet, dass** man mindestens ein verestertes Dihydroxyaceton-Derivat entsprechend der allgemeinen Formel I nach Anspruch 1 in Wasser löst, die Lösung auf eine Temperatur bringt, die gegenüber der Raumtemperatur erhöht ist, und die Lösung auf den menschlichen Körper aufträgt.

23. Verfahren nach Anspruch 22, **dadurch gekennzeichnet, dass** der menschliche Körper ganz oder teilweise in die Lösung eingetaucht wird.

24. Verfahren nach Anspruch 22, **dadurch gekennzeichnet, dass** zur gleichmäßigen Bräunung der menschliche Körper gleichmäßig ganz oder teilweise mit der erhitzten Lösung eingesprüht wird.

25. Verfahren nach mindestens einem der Ansprüche 22 bis 24, **dadurch gekennzeichnet, dass** auf Körperpartien, die nicht oder nur wenig gebräunt werden sollen, eine wasserabstoßende Zubereitung aufgetragen wird.

## Claims

1. Cosmetic or dermatological composition which comprises, in a cosmetically or dermatologically acceptable vehicle, at least one esterified dihydroxyacetone derivative having the general formula I 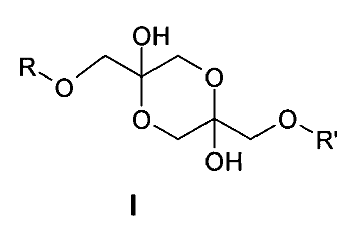 in which R and R' denote a hydrogen atom or a saturated or unsaturated, linear, branched or cyclic, optionally hydroxylated acyl radical having 2 to 25 carbon atoms, where R and R' may be identical or different on condition that they never simultaneously denote a hydrogen atom.

2. Composition according to Claim 1, furthermore comprising at least one compound capable of cleaving at least one ester bond.

3. Composition according to Claim 1 or 2, in which the acyl radical is a benzoyl radical, an alkylbenzoyl radical, an acylbenzoyl radical or a 2-hydroxy-2-phenylacetyl radical.

4. Composition according to at least one of the preceding claims, in which the acyl radical is hydroxylated.

5. Composition according to at least one of the preceding claims, in which the acyl radical has 3 to 18 carbon atoms and in which the esterified derivative is preferably selected from the group 2-oxopropyl 1,3-didodecanoate, 2-oxopropyl 1,3-dihexadecanoate and 2-oxopropyl 3-hexadecanoate.

6. Composition according to at least one of the preceding claims, in which the esterified derivative is present in a concentration in the range from 0.1 % by weight to 20% by weight, preferably from 0.5% by weight to 10% by weight, based on the total weight of the composition.

7. Composition according to Claim 2, in which the compound capable of cleaving at least one ester bond is a nucleophilic compound, preferably an amine, an anion or an enzyme, particularly preferably a hydroxylated amine, a carboxylate anion or a hydrolase.

8. Composition according to Claim 2 or 7, in which the compound capable of cleaving at least one ester bond is selected from the group 3-amino-1,2-propanediol, 2-amino-2-methyl-1,3-propanediol, 2-amino-2-methylpropanol, 2-amino-2-hydroxymethyl-1 ,3-propanediol, glucamine or N-methylglucamine, a lysine, arginine and a histidine.

9. Composition according to Claim 2, in which the compound capable of cleaving at least one ester bond is a lipase, an esterase or a protease, preferably a lipase.

10. Composition according to at least one of Claims 2 or 7 to 9, in which the compound capable of cleaving at least one ester bond is present in a concentration in the range from 0.1 % by weight to 30% by weight, preferably 0.5% by weight to 15% by weight, based on the total weight of the composition.

11. Composition according to at least one of Claims 2 or 7 to 10, in which the esterified derivative and the compound capable of cleaving at least one ester bond are packaged so as not to be in contact with one another.

12. Composition according to Claim 11, in which the esterified derivative and the compound capable of cleaving at least one ester bond are contained in a single packaging with two compartments.

13. Composition according to Claim 12, in which at least one of the esterified derivative and the compound capable of cleaving at least one ester bond is encapsulated.

14. Composition according to at least one of the preceding claims, **characterised in that** the composition comprises at least one fatty vehicle and at least one hydrophilic solvent, where the fatty vehicle present is preferably selected from one or more compounds of the ceramides, cholesterol, phospholipids, cholesteryl sulfate, cholesteryl phosphate, phosphatidylcholine, lecithin and/or empty liposomes, and the fatty vehicle is preferably present in a concentration of 0.1 to 10% by weight.

15. Composition according to at least one of the preceding claims, **characterised in that** the formulation comprises one or more UV filters.

16. Composition according to Claim 14, **characterised in that** the formulation comprises, as hydrophilic solvent, glycerol and/or propylene glycol, preferably in a concentration of 0.1 to 20% by weight.

17. Composition according to at least one of the preceding claims, **characterised in that** the formulation is a liquid, creamy, milky and/or gel-like bath additive, a bath tablet, a bath salt and/or a bath capsule.

18. Method for colouring the skin, which consists in applying thereto a composition according to at least one of Claims 1 to 17.

19. Method for colouring the skin according to Claim 18, with application at elevated temperature, in which the application temperature is preferably in the range between 25 and 60°C, more preferably between 30 and 55°C and particularly preferably between 37 and 50°C.

20. Method for colouring the skin according to at least one of Claims 18 or 19, **characterised in that** application takes place in a bathtub or whirlpool.

21. Method for colouring the skin according to at least one of the claims 18 or 19, **characterised in that** application to the skin takes place by spraying, preferably by means of a shower or spray gun.

22. Method of tanning the human body, **characterised in that** at least one esterified dihydroxyacetone derivative conforming to the general formula I according to Claim 1 is dissolved in water, the solution is brought to a temperature which is elevated relative to room temperature, and the solution is applied to the human body.

23. Method according to Claim 22, **characterised in that** all or part of the human body is immersed into the solution.

24. Method according to Claim 22, **characterised in that**, for even tanning, all or part of the human body is sprayed evenly with the heated solution.

25. Method according to at least one of Claims 22 to 24, **characterised in that** a water-repellent preparation is applied to body parts which are not to be tanned or are to be tanned only slightly.

## Revendications

1. Composition cosmétique ou dermatologique comprenant, dans un véhicule cosmétiquement ou dermatologiquement acceptable, au moins un dérivé de dihydroxyacétone estérifiée répondant à la formule générale I 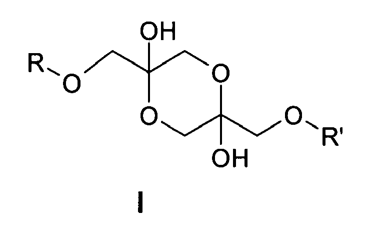 dans laquelle R et R' désignent un atome d'hydrogène ou un radical acyle saturé ou insaturé, linéaire, ramifié ou cyclique, et éventuellement hydroxylé, ayant de 2 à 25 atomes de carbone, où R et R' peuvent être identiques ou différents à condition qu'ils ne désignent jamais simultanément un atome d'hydrogène.

2. Composition selon la revendication 1, comprenant en outre au moins un composé capable de cliver au moins une liaison ester.

3. Composition selon la revendication 1 ou 2, dans laquelle le radical acyle est un radical benzoyle, un radical alkylbenzoyle, un radical acylbenzoyle ou un radical 2-hydroxy-2-phénylacétyle.

4. Composition selon au moins l'une des revendications précédentes, dans laquelle le radical acyle est hydroxylé.

5. Composition selon au moins l'une des revendications précédentes, dans laquelle le radical acyle contient de 3 à 18 atomes de carbone et dans laquelle le dérivé estérifié est sélectionné de préférence parmi le groupe constitué par le 1,3-didodécanoate de 2-oxopropyle, le 1,3-dihexadécanoate de 2-oxopropyle et le 3-hexadécanoate de 2-oxopropyle.

6. Composition selon au moins l'une des revendications précédentes, dans laquelle le dérivé estérifié est présent selon une concentration dans la plage de 0,1 % en poids à 20% en poids, de préférence de 0,5% en poids à 10% en poids, par rapport au poids total de la composition.

7. Composition selon la revendication 2, dans laquelle le composé capable de cliver au moins une liaison ester est un composé nucléophile, de préférence une amine, un anion ou une enzyme, de manière particulièrement préférée une amine hydroxylée, un anion carboxylate ou une hydrolase.

8. Composition selon la revendication 2 ou 7, dans laquelle le composé capable de cliver au moins une liaison ester est sélectionné parmi le groupe constitué par le 3-amino-1,2-propanediol, le 2-amino-2-méthyl-1,3-propanediol, le 2-amino-2-méthylpropanol, le 2-amino-2-hydroxyméthyl-1,3-propanediol, la glucamine ou la N-méthylglucamine, une lysine, une arginine et une histidine.

9. Composition selon la revendication 2, dans laquelle le composé capable de cliver au moins une liaison ester est une lipase, une estérase ou une protéase, de préférence une lipase.

10. Composition selon au moins l'une parmi les revendications 2 ou 7 à 9, dans laquelle le composé capable de cliver au moins une liaison ester est présent selon une concentration dans la plage de 0, 1 % en poids à 30% en poids, de préférence de 0,5% en poids à 15% en poids, par rapport au poids total de la composition.

11. Composition selon au moins l'une parmi les revendications 2 ou 7 à 10, dans laquelle le dérivé estérifié et le composé capable de cliver au moins une liaison ester sont conditionnés de façon à ne pas être en contact l'un avec l'autre.

12. Composition selon la revendication 11, dans laquelle le dérivé estérifié et le composé capable de cliver au moins une liaison ester sont contenus dans un seul conditionnement à deux compartiments.

13. Composition selon la revendication 12, dans laquelle au moins l'un parmi le dérivé estérifié et le composé capable de cliver au moins une liaison ester est encapsulé.

14. Composition selon au moins l'une des revendications précédentes, **caractérisée en ce que** la composition comprend au moins un véhicule gras et au moins un solvant hydrophile, où le véhicule gras présent est sélectionné de préférence parmi un ou plusieurs composés parmi les céramides, le cholestérol, les phospholipides, le sulfate de cholestéryle, le phosphate de cholestéryle, la phosphatidylcholine, la lécithine et/ou les liposomes vides, et le véhicule gras est présent de préférence à une concentration de 0,1 à 10% en poids.

15. Composition selon au moins l'une des revendications précédentes, **caractérisée en ce que** la formulation comprend un ou plusieurs filtres UV.

16. Composition selon la revendication 14, **caractérisée en ce que** la formulation comprend, comme solvant hydrophile, du glycérol et/ou du propylène glycol, de préférence selon une concentration de 0,1 à 20% en poids.

17. Composition selon au moins l'une des revendications précédentes, **caractérisée en ce que** la formulation est un additif de bain liquide, crémeux, laiteux et/ou sous forme de gel, un comprimé de bain, un sel de bain et/ou une capsule de bain.

18. Méthode de coloration de la peau, qui consiste à appliquer sur celle-ci une composition selon au moins l'une parmi les revendications 1 à 17.

19. Méthode de coloration de la peau selon la revendication 18, par une application à température élevée, dans laquelle la température d'application est de préférence dans la plage entre 25 et 60°C, plus préférablement entre 30 et 55°C et de manière particulièrement préférée entre 37 et 50°C.

20. Méthode de coloration de la peau selon au moins l'une parmi les revendications 18 ou 19, **caractérisée en ce que** l'application est réalisée dans une baignoire ou une baignoire à remous.

21. Méthode de coloration de la peau selon au moins l'une parmi les revendications 18 ou 19, **caractérisée en ce que** l'application sur la peau est réalisée par pulvérisation, de préférence au moyen d'une douche ou d'un pistolet de pulvérisation.

22. Méthode de bronzage du corps humain, **caractérisée en ce qu'**au moins un dérivé de dihydroxyacétone estérifiée conforme à la formule générale I selon la revendication 1 est dissous dans de l'eau, la solution est amenée à une température élevée par rapport à la température ambiante, et la solution est appliquée sur le corps humain.

23. Méthode selon la revendication 22, **caractérisée en ce qu'**on immerge la totalité ou une partie du corps humain dans la solution.

24. Méthode selon la revendication 22, **caractérisée en ce que**, pour un bronzage homogène, on pulvérise uniformément la solution chauffée sur la totalité ou une partie du corps humain.

25. Méthode selon au moins l'une parmi les revendications 22 à 24, **caractérisée en ce qu'**on applique une préparation hydrofuge aux parties du corps destinées à être peu ou pas bronzées.
